(19)

**Europäisches Patentamt**
**European Patent Office**
**Office européen des brevets**

(11) **EP 4 456 077 A1**

(12) **EUROPEAN PATENT APPLICATION**

(43) Date of publication:
**30.10.2024 Bulletin 2024/44**

(21) Application number: **23170271.3**

(22) Date of filing: **27.04.2023**

(51) International Patent Classification (IPC):
**G16C 20/30** (2019.01)    **G16C 20/70** (2019.01)
**G16C 60/00** (2019.01)

(52) Cooperative Patent Classification (CPC):
(C-Sets available)
**G16C 20/30; C08L 23/10; G06N 3/126;**
**G06N 20/00;** C08L 2207/20; **G16C 20/70;**
**G16C 60/00**                    (Cont.)

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(71) Applicant: **Borealis AG**
**1020 Vienna (AT)**

(72) Inventors:
• **TORRES, Juan Pablo**
**4021 Linz (AT)**

• **JERABEK, Michael**
**4021 Linz (AT)**
• **MILEVA, Daniela**
**4021 Linz (AT)**
• **KIEHAS, Florian**
**4040 Linz (AT)**
• **REITER, Martin**
**4040 Linz (AT)**

(74) Representative: **Ter Meer Steinmeister & Partner**
**Patentanwälte mbB**
**Nymphenburger Straße 4**
**80335 München (DE)**

(54) **MACHINE LEARNING FOR PROPERTY PREDICTION AND RECIPE RECOMMENDATION OF POLYOLEFIN-BASED POLYMER BLENDS**

(57)    The present invention relates to a computer-implemented method for predicting attributes of a polypropylene composition. The method includes training a fingerprinting model with polypropylene training compositions by mapping contents of ingredients of the polypropylene training compositions to analytical features of the polypropylene training compositions, feeding the trained fingerprinting model, as an input, with contents of ingredients of a polypropylene test composition, and receiving, as an output, analytical features of the polypropylene test composition. In this context, the analytical features comprise at least one feature characteristic of the amorphous fraction or the crystalline fraction of the polypropylene composition. Furthermore, the present invention provides a computer readable storage medium for storing computer program instructions defining the steps of the inventive method. Besides that, the present invention encompasses the use of analytical features comprising at least one feature characteristic of the amorphous fraction or the crystalline fraction of a polypropylene composition and contents of ingredients of polypropylene compositions for training a computer-implemented model.

Figure 1

(52) Cooperative Patent Classification (CPC): (Cont.)

C-Sets
**C08L 23/10, C08L 23/0815, C08K 3/013**

## Description

### Field of the invention

The present invention relates to a computer-implemented method for predicting attributes of a polypropylene composition. The method includes training a fingerprinting model with polypropylene training compositions by mapping contents of ingredients of the polypropylene training compositions to analytical features of the polypropylene training compositions, feeding the trained fingerprinting model, as an input, with contents of ingredients of a polypropylene test composition, and receiving, as an output, analytical features of the polypropylene test composition. In this context, the analytical features comprise at least one feature characteristic of the amorphous fraction or the crystalline fraction of the polypropylene composition. Furthermore, the present invention provides a computer readable storage medium for storing computer program instructions defining the steps of the inventive method. Besides that, the present invention encompasses the use of analytical features comprising at least one feature characteristic of the amorphous fraction or the crystalline fraction of a polypropylene composition and contents of ingredients of polypropylene compositions for training a computer-implemented model.

### Technical background

Polypropylene compositions are omnipresent. Polypropylene compositions including heterophasic propylene copolymers are, for example, widely used for automotive applications as they exhibit an excellent combination of stiffness and impact behaviour. Still, they need to be developed further, for example in view of the automotive industry's declared aim to reduce weight, on the one hand, and to include more and more recyclate materials, on the other hand.

Despite of the modifications that are necessary to these ends, new polypropylene compositions should have excellent chemical and physical properties. A high flowability, which is synonymous to a high melt flow rate, and a high dimensional stability after processing, which can be equated to a low initial coefficient of linear thermal expansion (CLTE), are desirable. Furthermore, optical properties, such as the surface appearance of the compositions, should not be impaired when changing the composition. Also, a good impact behaviour of a known polypropylene composition, such as a high notched Charpy impact strength and instrumented puncture resistance, should be maintained. Likewise, a high stiffness, recorded as high tensile modulus, should not be sacrificed. Hence, the development of new polypropylene compositions is governed by numerous constraints.

Some of the constraints can be met by tailoring the polypropylene virgin materials that are used for the compositions. Others can be targeted by introducing suitable amounts of other specific ingredients. Amongst others, such ingredients comprise polymeric ingredients, which can adapt the properties of the one or more polypropylenes contained in the compositions. Elastomeric polymers like plastomers are, for example, predestined to modify the properties of the elastomeric phase in a heterophasic propylene copolymer. Propylene homopolymers are suitable to influence the properties of the matrix phase in a heterophasic propylene copolymer. Apart from polymeric ingredients, fillers are valuable components to be included in new compositions. They can be used to endow any polypropylene composition with good impact strength and high stiffness.

Summarizing, several instruments for influencing the properties of a polypropylene composition are known.

Nevertheless, the development of new polypropylene compositions remains challenging. An appropriate recipe that is able to meet all constraints (typically a combination of mechanical, rheological and aesthetical constraints) needs to be identified. This requires a considerable amount of human expertise and experience. Moreover, it is commonplace to apply a trial-and-error approach, which includes producing, testing and adjusting candidate recipes in an iterative fashion. Therefore, the development of new compositions also ties up a considerable amount of resources, *i.e.* it is time-consuming, blocks analytical instruments and is cost intensive.

In view of the above, there is an apparent need for a computer-implemented method for predicting the attributes of a polypropylene composition.

Generally, computer-implemented methods for predicting material properties are known from the prior art.

WO 2022/0199971 A1, for instance, discloses a method for predicting the properties of a formulation. A candidate formulation recipe and one or more target properties and target property constraints are input into a trained machine learning model. As an output, the method delivers the predicted property of the candidate formulation and the predicted likelihood that the predicted property satisfies the one or more target properties and target property constraints. To that end, the machine learning model has been trained using historical training data, each element of the historical training data corresponding to a known formulation having a known feature representation. In the field of polymer compositions, the method is purported to be useful for the prediction of shelf-life times of compounded polymer blends and for the prediction of mechanical properties of rubber formulations for vehicle tires. However, there is no indication of how to apply the model to predict attributes of polypropylene compositions or of how to further improve the quality of the predictions.

Furthermore, US 10,955,362 B2 describes a computer-implemented method for determining polymer properties. To that end, a Raman spectrum of a polymer sample is obtained and polymer properties are computed by comparing the chemical and/or structural fingerprint to previously stored properties data using a machine learning tool. The algorithms of the machine learning tool are trained with historical Raman data and polymer properties of known samples measured in a laboratory with conventional methods. For a desired polymer property, a data set - including the measured polymer properties and the respective Raman spectral data is ingested into a database accessible via an application programming interface, API. The method provides reasonable predictions for polymer properties such as the MFR and the ethylene content. Yet, the method is not useful for predicting arbitrary attributes with a high accuracy. Also, it is relatively resource-intensive to obtain the required Raman spectra.

Consequently, it has been an objective of the present invention to provide an improved computer-implemented method, which is able predict attributes of polypropylene compositions with a high accuracy, in a minimum amount of time, at a minimum expense and based on data that can be obtained with reasonable experimental effort.

## Summary of the invention

The above identified objective is accomplished by the computer-implemented method according to independent claim 1 and the use of a feature characteristic of the amorphous fraction or the crystalline fraction of a polypropylene composition in machine learning according to claim 15. Advantageous embodiments may be derived from the dependent claims.

The computer-implemented method for predicting attributes of a polypropylene composition comprises at least three steps. In a first step (step Ia), a fingerprinting model is trained with polypropylene training compositions (hereinafter also "training phase"). This is done by mapping contents of ingredients of the polypropylene training compositions to analytical features of the polypropylene training compositions. In a second step (step Ib), the trained fingerprinting model is fed, as an input, with contents of ingredients of a polypropylene test composition. A third step of the method (step Ic) comprises receiving, as an output, analytical features of the polypropylene test composition, wherein the analytical features comprise at least one feature characteristic of the amorphous fraction or the crystalline fraction of the polypropylene composition. The second and third step may, therefore, also be referred to as the "prediction phase".

The attributes that are predicted by the computer-implemented method according to the present invention may be selected from the group consisting of analytical features, chemical properties, physical properties, recipes and combinations thereof.

Furthermore, the fingerprinting model may also be denoted a "fingerprinting machine learning algorithm" or a "first machine learning algorithm" or "Model I".

### Ingredients

The polypropylene training compositions and the polypropylene test composition comprise at least one polypropylene. The at least one polypropylene may be selected from the group consisting of propylene homopolymer, random propylene copolymer, heterophasic propylene copolymer and combinations or blends thereof. The heterophasic propylene copolymer may be a heterophasic propylene copolymer comprising a propylene random copolymer as a matrix phase (RAHECO) or a heterophasic propylene copolymer having a propylene homopolymer as matrix phase (HECO).

Furthermore, the polypropylene training compositions and/or the polypropylene test composition may comprise at least one further ingredient selected from the group consisting of impact modifier, filler and polyethylene. Moreover, additives may be present in the polypropylene training compositions and/or the polypropylene test composition as well, preferably in small amounts.

### Polypropylene

The at least one polypropylene may be a virgin polypropylene or a recycled polypropylene. Preferably, the polypropylene training compositions comprise from 10 to 100 wt.-%, more preferably from 30 to 100 wt.-%, of a recycled polypropylene, based on the total amount of the respective polypropylene training composition. The polypropylene test composition preferably comprises from 10 to 95 wt.-%, more preferably from 30 to 85 wt.-%, of a recycled polypropylene, based on the total amount of the polypropylene test composition.

A recycled polypropylene is the opposite of a virgin polypropylene.

A virgin polypropylene is a material that is newly produced, in particular from propylene and optionally other monomeric units such as alpha olefins. The term "virgin" may, furthermore, apply to a material, which has not yet completed a first use cycle and/or has not yet been recycled.

In contrast to that, a recycled polypropylene is preferably recovered from waste plastic material derived from post-consumer waste and/or post-industrial waste, preferably from waste plastic material derived only from post-consumer waste. Post-consumer waste refers to objects having completed at least a first use cycle (or life cycle), *i.e.* having already served their first purpose; while industrial waste refers to manufacturing scrap, which does not normally reach a consumer. The recycled polypropylene preferably comprises polypropylene in a content from 40 to 99.9 wt.-%, more preferably from 45 to 99.0 wt.-%, most preferably from 50 to 98.0 wt.-%.

In terms of analytic methods, virgin polypropylene and recycled polypropylene may be differentiated based on the absence or presence of contaminants, especially limonene, which may be detected according to the method described below in the example section. Other contaminants of recycled polypropylene may be fatty acids and/or paper and/or wood. Furthermore, virgin polypropylene and recycled polypropylene may be differentiated based on the black spot concentration, which can be determined according to the method described below in the example section. Further indicators of the presence of a recycled polypropylene are the color, in particular the L*, a* and b* values according to the CIELAB color space, and the amount of inorganic residues as determined according to the method described below in the example section.

Propylene homopolymer

The polypropylene training compositions and/or the polypropylene test composition may comprise at least one propylene homopolymer in an amount of 2.5 wt.-% or more, based on the total amount of the respective composition. Preferably, the polypropylene training compositions and the polypropylene test composition comprise from 2.5 to 30 wt.-%, more preferably from 5 to 25 wt.-%, of a propylene homopolymer.

The propylene homopolymer may be a visbroken or a non-visbroken propylene homopolymer. The visbroken propylene homopolymer may have been obtained by visbreaking according to any known visbreaking technology. However, it is preferred that a visbreaking agent is employed. Preferably, the visbreaking agent is a peroxide radical generator.

In a preferred embodiment, the at least one propylene homopolymer has a density determined according to ISO 1183-1 in the range of 800 to 1000 kg/m$^3$, preferably in the range of 850 to 950 kg/m$^3$.

In a further preferred embodiment, the at least one propylene homopolymer has a melt flow rate MFR$_2$ (230 °C, 2.16 kg, measured according to ISO 1133) in the range of 600 to 1300 g/10 min, preferably of 700 to 1250 g/10 min.

It is further preferred if the at least one propylene homopolymer has a melting temperature of at least 140 °C, preferably of at least 150 °C, more preferably in the range of 150 to 165 °C.

Preferably, the propylene homopolymer is free of phthalic compounds as well as their respective decomposition products. In another embodiment of the present invention, the propylene homopolymer has 2,1 erythro regio-defects of equal or below 0.4 mol-% determined by $^{13}$C-NMR spectroscopy, and/or a pentad isotacticity (mmmm) of more than 90.0 %, and/or a xylene cold soluble (XCS) content determined according to ISO 16152 (25 °C) of at least 1.8 wt.-%, and/or a crystallization temperature Tc of more than 110 °C.

Polypropylene homopolymers fulfilling the above specifications are known in the art and are commercially available.

Random propylene copolymer

The polypropylene training compositions and/or the polypropylene test composition may comprise at least one random copolymer, *i.e.* a random propylene copolymer, in an amount of 2.5 wt.-% or more, based on the total amount of the respective composition. Preferably, the polypropylene training compositions and the polypropylene test composition comprise from 2.5 to 30 wt.-% of a random propylene copolymer.

The term "random copolymer" has to be preferably understood according to IUPAC (Pure Appl. Chem., Vol. No. 68, 8, pp. 1591 to 1595, 1996).

The random propylene copolymer comprises monomers copolymerizable with propylene, for example comonomers such as ethylene and/or C$_4$ to C$_{12}$ $\alpha$-olefins, in particular ethylene and/or C$_4$ to C$_8$ $\alpha$-olefins, e.g. 1-butene and/or 1-hexene. Preferably the random propylene copolymer according to this invention comprises, especially consists of, monomers copolymerizable with propylene from the group consisting of ethylene, 1-butene and 1-hexene. In a preferred embodiment, the random propylene copolymer comprises units derivable from ethylene and propylene only.

The comonomer content of the random propylene copolymer is preferably not more than 7.0 wt.-%, more preferably not more than 6.0 wt.-%, still more preferably in the range of 1.0 to 7.0 wt.-%, yet more preferably in the range of 1.5 to 6.0 wt.-%, still more preferably in the range of 1.5 to 5.5 wt.-%, like in the range of 2.0 to below 5.0 wt.-%.

Heterophasic propylene copolymer

The polypropylene training compositions and/or the polypropylene test composition may comprise at least one heterophasic propylene copolymer in an amount of 5 wt.-% or more, based on the total amount of the respective composition. Preferably, the polypropylene training compositions and the polypropylene test composition comprise from 5 to 98 wt.-%, more preferably from 5 to 60 wt.-%, of a heterophasic propylene copolymer.

The heterophasic propylene copolymer may comprise a propylene homopolymer matrix phase (HECO) or a random propylene copolymer matrix phase (RAHECO) and an elastomeric propylene copolymer which is dispersed within said matrix phase. Preferably, the heterophasic propylene copolymer has a melt flow rate $MFR_2$ (at 230 °C) of at least 5 g/10 min, more preferably in a range from 5 to 300 g/10 min, most preferably in a range from 5 to 120 g/10 min.

Heterophasic propylene copolymers are well known in the art and may be produced by compounding ("compound grade") or, which is more preferred, in a multistage polymerization process ("reactor grade"). The compounding production procedure includes melt mixing isotactic polypropylene with a certain amount of a separately produced elastomer, such as an ethylene-propylene rubber (EPR) or an ethylene-propylene-diene elastomer (EPDM). The production in a multistage polymerization process uses two or more sequential reactors and includes first polymerizing propylene to polypropylene in the first one or two reactors and then polymerizing elastomer components, such as ethylene and propylene, in the presence of the polypropylene in the subsequent reactor. A preferred multistage process for preparing the heterophasic propylene copolymer is a "loop-gas phase"-process (known as BORSTAR technology) described e.g. in patent literature, such as in EP 0 887 379, WO 92/12182 WO 2004/000899, WO 2004/111095, WO 99/24478, WO 99/24479 or WO 00/68315.

Also suitable is a slurry-gas phase process (e.g. the Spheripol process).

A suitable catalyst for the polymerization of the heterophasic propylene copolymer is any stereospecific catalyst for propylene polymerization, which is capable of polymerizing and copolymerizing propylene and comonomers at a temperature of 40 to 110 °C and at a pressure from 10 to 100 bar. Therefore, Ziegler-Natta as well as metallocene catalysts are suitable catalysts.

In one embodiment, the heterophasic propylene copolymer consists of the propylene homopolymer or random propylene copolymer matrix phase and the elastomeric propylene copolymer being dispersed within the matrix phase. In another embodiment, the heterophasic propylene copolymer may contain additives. However, the heterophasic propylene copolymer may not contain other polymers in an amount exceeding 10 wt.-%, preferably 5 wt.-%, more preferably exceeding 3 wt.-%, like exceeding 1 wt.-%, based on the total amount of the heterophasic propylene copolymer. One polymer which may be present in such low amounts is a polyethylene which is a reaction product obtained by the preparation of the heterophasic propylene copolymer.

Preferably, the propylene content in the heterophasic propylene copolymer is 82.0 to 97.0 wt.-%, more preferably 86.0 to 95.0 wt.-%, based on the total amount of the heterophasic propylene copolymer, more preferably based on the amount of the polymer components of the heterophasic propylene copolymer, yet more preferably based on the amount of the propylene homopolymer or random propylene copolymer matrix phase and the elastomeric propylene copolymer together. Accordingly, the comonomer content, preferably ethylene content, is in the range of 3.0 to 18.0 wt.-%, more preferably in the range of 5.0 to 14.0 wt.-%, based on the total amount of the heterophasic propylene copolymer, more preferably based on the amount of the polymer components of the heterophasic propylene copolymer, yet more preferably based on the amount of the propylene homopolymer or random propylene copolymer matrix phase and the elastomeric propylene copolymer together.

The matrix phase of the heterophasic polypropylene can be made of the propylene homopolymer (HECO) or random propylene copolymer (RAHECO) only but can also comprise additional polymers, in particular polymers which can be blended homogeneously with the propylene homopolymer or random propylene copolymer and together form a continuous phase which can act as a matrix. In a preferred embodiment, at least 90 wt.-% of the matrix, more preferably at least 95 wt.-% of the matrix are made of the propylene homopolymer or random propylene copolymer. Even further preferred, the matrix consists of the propylene homopolymer or random propylene copolymer.

In case the matrix phase is a random propylene copolymer, it comprises monomers copolymerizable with propylene, for example comonomers such as ethylene and/or $C_4$ to $C_{12}$ alpha-olefins, in particular ethylene and/or $C_4$ to $C_{10}$ alpha-olefins, e.g. 1-butene and/or 1-hexene. Preferably the random propylene copolymer comprises, especially consists of, monomers copolymerizable with propylene selected from the group consisting of ethylene, 1-butene and 1-hexene. More specifically, the random propylene copolymer comprises - apart from propylene - units derivable from ethylene and/or 1-butene. In a preferred embodiment, the random propylene copolymer comprises units derivable from ethylene and propylene only. The comonomer content in the random propylene copolymer is preferably in the range of 0.5 to 10.0 wt.-%, still more preferably in the range of more than 0.5 to 7.0 wt.-%.

In case the matrix phase is a propylene homopolymer, the propylene homopolymer is preferably isotactic. Accordingly, the propylene homopolymer may have a rather high pentad concentration, *i.e.* more than 90 %, more preferably more than 92 %, still more preferably more than 93 % and yet more preferably more than 95 %, like at least 97 %.

The propylene homopolymer or random propylene copolymer matrix phase may have a XCS content in a broad range, *i.e.* up to 6.0 wt.-%, based on the total amount of propylene homopolymer or random propylene copolymer matrix phase. Accordingly the propylene homopolymer or copolymer matrix phase may have a XCS content in the range from 0.3 to 6.0 wt.-%, e.g., from 0.5 to 5.5 wt.-%, based on the amount of the propylene homopolymer or random propylene copolymer matrix phase. In case the matrix phase is a propylene homopolymer, it has a XCS content in the range from 0.5 to 4.5 wt.-%, more preferably in the range from 0.5 to 4.0 wt.-%, still more preferably from 0.8 to 3.5 wt.-%, based on the total amount of the propylene homopolymer matrix phase.

According to one embodiment of the present invention, the propylene homopolymer or random propylene copolymer matrix phase has a melt flow rate $MFR_2$ (at 230 °C) of 10 to 300 g/10 min, preferably in the range from 25 to 150 g/10 min, more preferably in the range from 30 to 100 g/10 min.

It is further preferred that the propylene homopolymer or random propylene copolymer forming the matrix phase has a weight average molecular weight, Mw, from 100,000 to 400,000 g/mol, preferably from 150,000 to 350,000, more preferably from 175,000 to 300,000 g/mol. The molecular weight distribution (MWD) of the propylene homopolymer or random propylene copolymer forming the matrix phase is at least 2.8, more preferably at least 3.0, e.g., at least 3.3. In a preferred embodiment, the MWD is preferably from 2.8 to 10.0, still more preferably from 3.0 to 8.0.

In addition to the propylene homopolymer or random propylene copolymer forming the matrix phase, the heterophasic propylene copolymer comprises an elastomeric propylene copolymer, which is dispersed within said matrix phase.

According to one embodiment, the elastomeric propylene copolymer comprises monomers copolymerizable with propylene, for example, comonomers such as ethylene and/or $C_4$ to $C_{12}$ alpha-olefins, preferably ethylene and/or $C_4$ to $C_{10}$ alpha-olefins, *e.g.* 1-butene and/or 1-hexene. Preferably the elastomeric propylene copolymer comprises, especially consists of, monomers copolymerizable with propylene from the group consisting of ethylene, 1-butene and 1-hexene. More specifically the elastomeric propylene copolymer comprises - apart from propylene - units derivable from ethylene and/or 1-butene. Thus, in an especially preferred embodiment the elastomeric propylene copolymer phase comprises units derivable from ethylene and propylene only.

In case the matrix phase of the heterophasic propylene copolymer is formed by a random propylene copolymer, it is preferred that the comonomer(s) of the random propylene copolymer and the elastomeric propylene copolymer are the same.

The properties of the elastomeric propylene copolymer phase mainly influence the XCS content of the heterophasic polymer propylene. Thus, according to the present invention the XCS fraction of heterophasic polymer propylene may be regarded as the fraction of the elastomeric propylene copolymer in the heterophasic propylene.

According to one embodiment of the present invention, the amount of the elastomeric propylene copolymer, *i.e.* of the XCS fraction, of the heterophasic propylene is in the range from 12 to 50 wt.-%, preferably in the range from 12 to 40 wt.-%, and more preferably in the range from 12 to 38 wt.-%, based on the total amount of the heterophasic propylene.

According to one embodiment of the present invention, the elastomeric propylene copolymer has a balanced weight average molecular weight. Small particles are formed in case the matrix and the elastomeric phase have similar molecular weight. Small particles are generally preferred, because this improves the overall properties of the heterophasic system.

The XCS fraction of the heterophasic polymer composition, may have an intrinsic viscosity (iv) in the range from 1.5 to 8.0 dL/g, more preferably in the range from 2.0 to 7.0 dL/g.

According to one embodiment of the present invention, the comonomer content, more preferably ethylene content, of the elastomeric propylene copolymer is equal or less than 75 wt.-%, preferably in the range of 15 to 75 wt.-%, more preferably in the range of 20 to 50 wt.-%, and most preferably in the range of 25 to 43 wt.-%, based on the total amount of the elastomeric propylene copolymer. Accordingly, it is appreciated that the propylene content of the elastomeric propylene copolymer, is equal or more than 25 wt.-%, preferably in the range from 25 to 85 wt.-%, more preferably in the range from 50 to 80 wt.-%, and most preferably in the range from 60 to equal or less than 75 wt.-%, based on the total amount of the elastomeric propylene copolymer.

Impact Modifier

The polypropylene training compositions and/or the test composition may further comprise at least one impact modifier in an amount of 2.5 wt.-% or more, based on the total amount of the respective composition. Preferably, the polypropylene training compositions and the polypropylene test composition comprise from 2.5 to 60 wt.-%, more preferably from 5 to 50 wt.-%, of an impact modifier.

The impact modifier may be selected from the group consisting of polyolefin elastomer, polyolefin plastomer, high-density polyethylene having high impact strength and combinations thereof. The polyolefin elastomer may be an elastomeric polyolefin copolymer.

Suitable elastomers may be ethylene/propylene copolymers with different ethylene/propylene ratio (C2/C3 or C3/C2 elastomers), ethylene/butene copolymers (C2/C4 elastomers), ethylene/octene copolymers (C2/C8 elastomers), grafted ethylene elastomers (such as maleic anhydride grafted ethylene elastomers) or C2/C3 and C2/C4 block copolymers, in particular an ethylene based 1-octene elastomer. Commercially available C2C8 impact modifiers are marketed under the trademarks Engage, Queo, Exact, Tafmer, Infuse and the like. As the maleic anhydride grafted ethylene elastomer commercially available impact modifiers marketed under the trademark name Fusabond may be used.

Suitable high-density polyethylenes having high impact strength have a density measured according to ISO 1183 of 945 to 960 kg/m$^3$ and/or a tensile impact strength at 23 °C measured according to ISO 8256/1A of 70 to 90 kJ/m$^2$. An example for a commercially available high-density polyethylene having high impact strength is MB7541 available from Borealis AG.

In a preferred embodiment, the impact modifier preferably has an MFR$_2$ (2.16 kg, 190 °C) of 1 to 20 g/10 min, more preferably 1 to 10 g/10 min. The impact modifier further preferably has an MFR$_2$ (2.16 kg, 230 °C) of 2 to 12 g/10 min.

Filler

The polypropylene training compositions and/or the test composition may further comprise at least one filler in an amount of 2.5 wt.-% or more, based on the total amount of the respective composition. Preferably, the polypropylene training compositions and the polypropylene test composition comprise from 2.5 to 30 wt.-%, more preferably from 5 to 25 wt.-%, of a filler.

Fillers may be particulate fillers, fibrous fillers or a combination thereof. The filler may be non-polymeric. For example, the filler may be a mineral filler, preferably an anisotropic mineral filler. Accordingly, the mineral filler may be selected from the group consisting of talc, mica, wollastonite, kaolinite, montmorillonite and mixtures thereof. Most preferably, the mineral filler is talc.

The mineral filler may have a defined particle size distribution. For example, the filler may have a particle size distribution d$_{95}$, as measured by sedimentation, in the range of 1 to 20 $\mu$m, preferably 5 to 15 $\mu$m. Furthermore, the filler may have a median particle size d$_{50}$, as measured by sedimentation, in the range of 0.5 $\mu$m to 5.0 $\mu$m, more preferably of 0.8 to 4.0 $\mu$m, most preferably 1.0 to 2.5 $\mu$m. Alternatively, the filler may have a particle size distribution d$_{95}$, as measured by laser diffraction, in the range of 1 to 50 $\mu$m, preferably 5 to 40 $\mu$m. Furthermore, the filler may have a median particle size d$_{50}$, as measured by laser diffraction, in the range of 2.0 $\mu$m to 15.0 $\mu$m, more preferably of 3.0 to 12.0 $\mu$m.

Also, the mineral filler may have a defined surface area as measured according to the commonly known BET method with N$_2$ gas as analysis adsorptive. The surface area may be more than 5 m$^2$/g, preferably more than 10 m$^2$/g, more preferably more than 12 m$^2$/g, still yet more preferably in the range of 5 to 25 m$^2$/g, like 10 to 20 m$^2$/g.

Additives

The polypropylene training compositions and the test composition may additionally contain additives.

The term "additives" does not include the filler, in particular the mineral filler selected from the group consisting of mica, wollastonite, kaolinite, montmorillonite, talc and mixtures thereof. In other words, the filler, in particular the mineral filler selected from the group consisting of talc, mica, wollastonite, kaolinite, montmorillonite and mixtures thereof, is not regarded as an additive.

Instead, the additives may be non-polymeric additives and/or polymeric additives. For example, the additives may be selected from the group consisting of acid scavengers, antioxidants, colorants, light stabilizers, plasticizers, nucleating agents, slip agents, antiscratch agents, dispersing agents, processing aids, lubricants, pigments, antistatic agent, and the like. Preferably, the amount of additives excluding the filler shall not exceed 7 wt.-%, more preferably shall not exceed 5 wt.-%, like not more than 2.5 wt.-%.

Analytical Features

The analytical features that are central to the training phase and the prediction phase comprise at least one feature characteristic of the amorphous fraction or the crystalline fraction of the respective polypropylene composition.

Preferably, the at least one feature characteristic of the amorphous fraction or the crystalline fraction is selected from the group consisting of an amorphous fraction percentage, a crystalline fraction percentage, a ratio of amorphous fraction percentage : crystalline fraction percentage, an ethylene content in the amorphous fraction, an ethylene content in the crystalline fraction, a ratio of ethylene content in the amorphous fraction : ethylene content in the crystalline fraction, an intrinsic viscosity in the amorphous fraction, an intrinsic viscosity in the crystalline fraction, and a ratio of intrinsic viscosity in the amorphous fraction : intrinsic viscosity in the crystalline fraction.

These features may be measured according to ISO 16152:2005 or ASTM D5492-17 by separating the amorphous fraction of the polypropylene composition based on the solubility in xylene at 25 °C. The fraction is quantified by weighing. The ethylene content of the XCS fraction and the xylene cold-insoluble (XCI) fraction may be analyzed by Fourier transform infrared (FTIR).

However, it is preferred when the analytical features of the polypropylene training compositions are obtained by an analysis with the CRYSTEX QC from Polymer Char (Valencia, Spain). A schematic representation of the CRYSTEX QC instrument is presented in Del Hierro, P.; Ortin, A.; Monrabal, B.; Soluble Fraction Analysis in polypropylene, The Column, February 2014. During the analysis with the CRYSTEX QC, a crystalline fraction and a soluble fraction are extracted by means of a solvent different from xylene, namely by use of 1,2,4-trichlorobenzene (TCB) or 1,2-ortho dichlorobenzene. The fractions are quantified and are examined with respect to the monomer and comonomer content as well as the intrinsic viscosity. The crystalline fraction represents the crystalline part of the polypropylene composition. The soluble fraction represents the amorphous part of the polypropylene composition.

The analytical features may further comprise at least one of a total ethylene weight fraction and an intrinsic viscosity of the respective polypropylene composition. These properties are, by default, also measured in an analysis with CRYSTEX QC, at least when these properties relate to the polypropylene training compositions. Therefore, the properties are available alongside the features characteristic of the amorphous fraction or the crystalline fraction without additional experimental effort.

Fingerprinting Model

In the training phase, the fingerprinting model maps the contents of ingredients of the polypropylene training compositions, for example the weight fractions of the ingredients, to analytical features of the polypropylene training compositions. The fingerprinting model is a machine learning algorithm. As an alternative to weight fractions, the model may operate with mole fractions of the ingredients.

From a technical point of view, model inputs and/or outputs may be in a vector form.

A first vector may comprise values representing the weight fractions of each of the ingredients of a polypropylene training composition. The first vector may have $n$ elements in total, if $n$ possible ingredients have been foreseen during the setup of the method. Should a particular ingredient not be part of the specific polypropylene training composition, the value for this element of the vector is set to "0". Hence, it may be understood that $n$ is a natural number, preferably a number from 2 to 100, more preferably from 2 to 80, in particular from 3 to 50.

A second vector may contain the analytical features of a polypropylene training composition. The second vector may have $m$ elements in total, with $m$ being a number from 1 to 20, preferably 2 to 10, in particular 8. If $m$ is at least 6, it is preferred that the at least 6 elements of the second vector contain values for a content of the amorphous fraction (sol) or the content of the crystalline fraction (cr), an ethylene content in the amorphous fraction (c2sol), an ethylene content in the crystalline fraction (c2cr), an intrinsic viscosity of the amorphous fraction (ivsol), an intrinsic viscosity of the crystalline fraction (ivcr), and a ratio of intrinsic viscosity of the amorphous fraction : intrinsic viscosity of the crystalline fraction (ivratio). If $m$ is 8, the 8 elements of the second vector may additionally contain values for the total ethylene weight fraction (c2) and the intrinsic viscosity (iv).

The values for the abovementioned analytical features are preferably experimental values, more preferably the results obtained by an analysis with CRYSTEX QC.

The fingerprinting model may be a regression-based algorithm. The regression-based algorithm preferably comprises a linear regression and, optionally, an additional quantile regression.

Preferably, the fingerprinting machine learning algorithm makes use of three regression-based algorithms in parallel, namely:

- a linear regression (LR), which maps the first vector containing the weight fraction of each ingredient to the second vector,

- a quantile regression (QR-U): which maps the first vector containing the weight fraction of each ingredient to a vector containing an upper quantile of each analytical feature, and

- a quantile regression (QR-L): which maps the first vector containing the weight fraction of each ingredient to a vector containing a lower quantile of each analytical feature.

The upper quantile may be chosen to be between 0.9 and 0.99, preferably between 0.93 and 0.97, in particular between 0.94 and 0.96. The lower quantile may be chosen to be between 0.01 and 0.1, preferably between 0.03 and 0.07, in particular between 0.04 and 0.06.

The use of the quantile regression allows to quantify the accuracy of the method.

Property Predicting Model

The computer-implemented method according to the present invention may further comprise use of a property predicting model. The property predicting model may also be denoted a "fingerprinting machine learning algorithm" or a "second machine learning algorithm". Moreover, the combination of the fingerprinting model and the property predicting model may be referred to as "Model II".

More specifically, the computer-implemented method according to the present invention may further comprise the following steps: training (step IIa) a property predicting model with polypropylene training compositions by mapping analytical features and at least contents of non-polymeric ingredients of polypropylene training compositions to at least one property of the polypropylene training compositions; feeding (step IIb) the trained property predicting model, as an input, with analytical features of the polypropylene test composition as received in step Ic) and at least contents of non-polymeric ingredients of the polypropylene test composition; receiving (step IIc), as an output, at least one property of the polypropylene test composition, wherein the at least one property is selected from the group consisting of chemical and physical properties.

Preferably, the computer-implemented method according to the present invention further comprises the following steps: training (step IIa) a property predicting model with polypropylene training compositions by mapping analytical features and contents of ingredients of the polypropylene training compositions to at least one property of the polypropylene training compositions; feeding (step IIb) the trained property predicting model, as an input, with analytical features of the polypropylene test composition as received in step Ic) and contents of ingredients of the polypropylene test composition; receiving (step IIc), as an output, at least one property of the polypropylene test composition, wherein the at least one property is selected from the group consisting of chemical and physical properties. The contents of ingredients of the polypropylene test composition may preferably be the same as the ones in step Ib).

In a preferred embodiment, the chemical and physical properties are selected from the group consisting of mechanical properties, rheological properties, processing properties and surface properties.

In a further preferred embodiment, the chemical and physical properties comprise at least one of the following properties:

- Young's modulus, preferably at room temperature (E-23) or at -30 °C (E-m30);

- Yield stress, preferably at room temperature (s-23) or at -30 °C (s-m30);

- Notched Charpy impact strength, preferably at room temperature (Charpy-23) or at -20 °C (Charpy-m20);

- Impact puncture energy, preferably at room temperature (ipt-23) or at -30 °C (ipt-m30);

- Coefficient of linear thermal expansion, preferably in flow direction (CLTE-MD) or in transverse direction (CLTE-TD);

- Melt flow rate (MFR), preferably at 2.16 kg and 230 °C ($MFR_2$) or at 21.6 kg and 230 °C ($MFR_{21}$);

- Shrinkage, preferably after injection in the flow direction (shri_sec_avg_flow) and/or perpendicular to the flow direction (shri_sec_avg_trans);

- a value indicative of tiger stripes, preferably after production of an injection mould with a filling time of 3.0 s (mse-3).

Among the above properties, the value indicative of tiger stripes is a value indicative of surface defects, which are known as "tiger stripes" or "flow marks". Such defects are a common problem for surface quality or visual appearance in plastic industry. Tiger stripes, as known in the plastic industry, describe a visible periodic inhomogeneity in surface gloss. Mostly these are alternating dull (or rough) and glossy (or smooth) areas on the surface of an injection moulded or extruded plastic part, the surface of which should be glossy (or smooth) all over. Accordingly, the value indicative of tiger stripes may be the predicted mean square error of a color variation on the surface of the injected or extruded polypropylene test composition.

The chemical and physical properties may alternatively or additionally include scaled versions of the aforementioned properties, preferably scaled versions obtained after a hyperbolic-tangent transformation or a logarithmic transformation.

Generally, scaling is a technique that can be used to introduce non-linearity in the model. This may significantly improve accuracy. Preferably, scaling is done based on subject matter domain knowledge. Subject matter domain knowledge may include the knowledge about cause-effect relationships. For example, it may include the awareness that impact strength can exhibit a step-like behaviour as a function of the impact modifier content. By scaling the respective property accordingly, the ML algorithm can be "informed" about this relationship. In the example of the impact strength, a hyperbolic-tangent as shown in Formula (1) below can be used as a function to suitably represent the step-like behaviour. If so, $y$ is equal to *charpy-23* or *charpy-m20.*

$$y_{scaled} = \tanh(y) \qquad (1)$$

Other properties are instead, more preferably scaled with a natural or decimal logarithmic transformation.

For an exemplary set of properties, appropriate scaling functions are shown in table 1 below.

*Table 1*

| hyperbolic tangent | logarithmic |
|---|---|
| charpy-23 | E-23 |
| charpy-m20 | E-m30 |
| ipt-23 | s-23 |
| ipt-m20 | s-m20 |
| mse-3 | CLTE-TD |
|  | CLTE-MD |
|  | MFR |
|  | shri_sec_avg_flow |

Young's modulus may also be referred to as tensile modulus and may be determined according to ISO 527-1:2012/ISO527-2:2012. According to the same standard, also the yield stress may be measured. Notched Charpy impact strength may be determined according to ISO 179-2:2020. Impact puncture energy may be determined by means of an instrumented falling weight test according to ISO 6603-2. The coefficient of linear thermal expansion may be determined according to ISO 11359-2:1999. The melt flow rate, *e.g.* $MFR_2$, may be determined at 230 °C based on ISO 1133. Shrinkage and the value indicative of the tiger stripes may be measured according to the respective methods as outlined below in the example section.

The property predicting model may be a Gaussian Process Regression (GPR). The Gaussian Process Regression is preferably defined by a kernel function. Suitable kernel functions can be derived from Pedregosa et al, "Gaussian Processes" in the Scikit-learn documentation.

Optimization Algorithm

The computer-implemented method may further comprise an optimization algorithm.

The optimization algorithm may be for predicting at least one recipe of a polypropylene composition, which comes closest to at least one predetermined target property. Preferably, the optimization algorithm is a genetic algorithm for predicting at least one recipe of a polypropylene composition, which comes closest to at least one predetermined target property.

The combination of the fingerprinting model, the property predicting model and the optimization algorithm may hereinafter be referred to as "Model III".

The optimization algorithm preferably comprises the following steps: receiving (step IIIa) at least one target property as an input, comparing (step IIIb) the at least one property of the polypropylene test composition as received in step IIc) with the at least one target property, and when the comparison fulfils a predetermined stopping criterion, receiving (step IIIc), as an output, the contents of ingredients of the polypropylene test composition in the form of a recipe.

If a genetic algorithm is used as the optimization algorithm, a non-dominated sorting based multi-objective evolutionary algorithm (NSGAII) receiving more than one target property as an input may be used.

Controlling Step

The output of the computer-implemented method according to the present invention may be used for controlling a process in a development of application-tailored polypropylene composition. For example, if the polypropylene test composition is a candidate composition which is suspected to satisfy the demands of a target application, the chemical or physical property which is output by Model II, i.e. in step IIc), may bring clarity. Likewise, the output obtained in step Ic) may be used to determine whether the polypropylene test composition is taken to the next research stage.

Storage medium

In another aspect, the present invention further provides a non-transitory computer readable storage medium for tangibly storing computer program instructions capable of being executed by a processor, the computer program instructions defining the steps of the method as described above.

However, the present invention also includes further means for storage and execution of the computer-implemented method as described above. For example, the invention extends to methods and apparatuses which perform the method described above, including data processing systems, which perform the method and computer readable media containing instructions which when executed on data processing systems cause the systems to perform the method.

Non-volatile memory can be a local device coupled directly to the rest of the components in the data processing system. A non-volatile memory that is remote from the system, such as a network storage device coupled to the data processing system through a network interface such as a modem or Ethernet interface, can also be used.

In the present disclosure, some functions and operations are described as being performed by or caused by software code to simplify description. However, such expressions are also used to specify that the functions result from execution of the code/instructions by a processor, such as a microprocessor.

Alternatively, or in combination, the functions and operations as described here can be implemented using special purpose circuitry, with or without software instructions, such as using Application-Specific Integrated Circuit (ASIC) or Field-Programmable Gate Array (FPGA). Embodiments can be implemented using hardwired circuitry without software instructions, or in combination with software instructions. Thus, the techniques are limited neither to any specific combination of hardware circuitry and software, nor to any particular source for the instructions executed by the data processing system.

While one embodiment can be implemented in fully functioning computers and computer systems, various embodiments are capable of being distributed as a computing product in a variety of forms and are capable of being applied regardless of the particular type of machine or computer-readable media used to actually effect the distribution.

At least some aspects disclosed can be embodied, at least in part, in software. That is, the techniques may be carried out in a computer system or other data processing system in response to its processor, such as a microprocessor, executing sequences of instructions contained in a memory, such as ROM, volatile RAM, non-volatile memory, cache or a remote storage device.

Routines executed to implement the embodiments may be implemented as part of an operating system or a specific application, component, program, object, module or sequence of instructions referred to as "computer programs." The computer programs typically include one or more instructions set at various times in various memory and storage devices in a computer, and that, when read and executed by one or more processors in a computer, cause the computer to perform operations necessary to execute elements involving the various aspects.

A machine-readable medium can be used to store software and data which when executed by a data processing system causes the system to perform various methods. The executable software and data may be stored in various places including for example ROM, volatile RAM, non-volatile memory and/or cache. Portions of this software and/or data may be stored in any one of these storage devices. Further, the data and instructions can be obtained from centralized servers or peer to peer networks. Different portions of the data and instructions can be obtained from different centralized servers and/or peer to peer networks at different times and in different communication sessions or in a same communication session. The data and instructions can be obtained in entirety prior to the execution of the applications. Alternatively, portions of the data and instructions can be obtained dynamically, just in time, when needed for execution. Thus, it is not required that the data and instructions be on a machine-readable medium in entirety at a particular instance of time.

Examples of computer-readable media include but are not limited to non-transitory, recordable, and non-recordable type media such as volatile and non-volatile memory devices, Read Only Memory (ROM), Random Access Memory (RAM), flash memory devices, floppy and other removable disks, magnetic disk storage media, optical storage media (e.g., Compact Disk Read-Only Memory (CD ROM), Digital Versatile Disks (DVDs), etc.), among others. The computer-readable media may store the instructions.

The instructions may also be embodied in digital and analog communication links for electrical, optical, acoustic or other forms of propagated signals, such as carrier waves, infrared signals, digital signals, etc. However, propagated signals, such as carrier waves, infrared signals, digital signals, etc. are not tangible machine readable medium and are not configured to store instructions.

In general, a machine-readable medium includes any mechanism that provides (i.e., stores and/or transmits) information in a form accessible by a machine (e.g., a computer, network device, personal digital assistant, manufacturing tool, any device with a set of one or more processors, etc.).

In various embodiments, hardwired circuitry may be used in combination with software instructions to implement the techniques. Thus, the techniques are neither limited to any specific combination of hardware circuitry and software nor to any source for the instructions executed by the data processing system.

Use

In another aspect, the present invention relates to the use of analytical features comprising at least one feature characteristic of the amorphous fraction or the crystalline fraction of a polypropylene composition and contents of ingredients of polypropylene compositions for training a computer-implemented model.

**Brief Description of the Drawings**

Embodiments of the present invention are illustrated by way of example and are not limited by the figures of the accompanying drawings in which identical references represent similar elements.

Fig. 1 shows the steps of the computer-implemented method according to the present invention in an embodiment including only the fingerprinting model (*i.e.* Model I);

Fig. 2 shows the steps involved in the training phase and the prediction phase of Model II;

Fig. 3 shows the method steps that are executed when running Model III;

Fig. 4 (a) - (h) show prediction accuracy plots of Model I for several analytical features;

Fig. 5 (a) - (h) show prediction accuracy plots of Model II for several chemical and physical properties in scaled versions after a hyperbolic-tangent transformation;

Fig. 6 (a) - (e) show prediction accuracy plots of Model II for several chemical and physical properties in scaled versions after a logarithmic transformation;

Figure 7 (a) - (c) show the Pareto front predicted by Model III and projected in the different possible planes for three target material properties.

**Detailed Description of the Drawings**

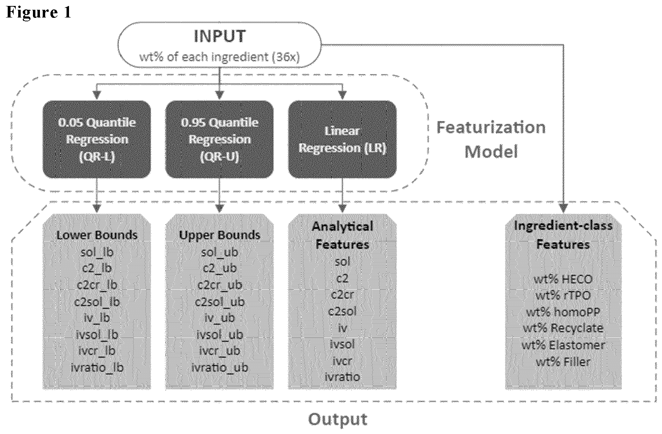

Figure 1 is a flowchart illustrating the operation of an exemplary Model I in the prediction phase. As can be inferred from the flowchart, a maximum of 36 ingredients have been foreseen for the polypropylene test composition during the setup of exemplary Model I (cf. "36x" in Fig. 1). Accordingly, trained Model I is fed, as an input, with a vector comprising values representing the weight fractions of the 36 possible ingredients (also denoted "ingredient-class features vector"). As an output, three vectors are received: One vector (also denoted "analytical feature vector") comprises the predicted values of eight analytical features (sol, c2, c2cr, c2sol, iv, ivsol, ivcr, ivratio) of the polypropylene test composition. The other two vectors contain the predicted 0.95 and 0.05 quantiles of said analytical features. Besides that, the input ingredient-class features vector, comprising values representing the weight fractions of the 36 possible ingredients of the polypropylene test composition, is treated as another output. When the model provides the analytical feature vector and the ingredient-class features vector as an output, it provides a suitable interface for a communication with the property predicting model and can be combined with the latter to provide Model II as described below.

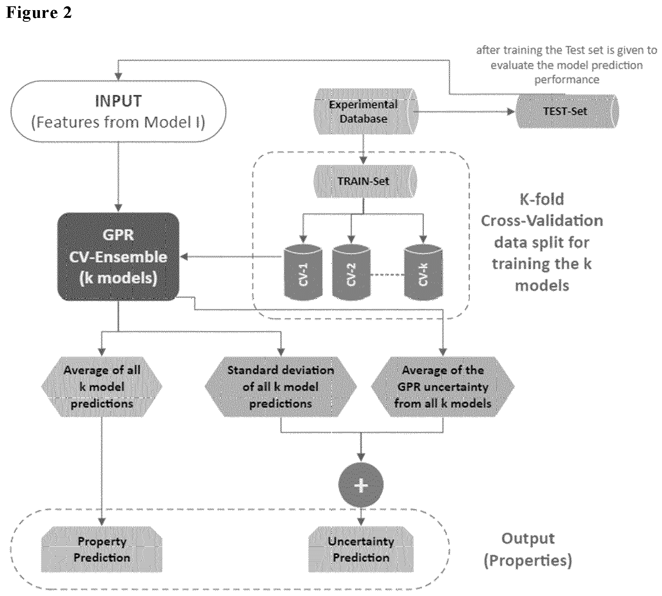

Figure 2 shows a flowchart of Model II. The features from Model I, *i.e.* the vectors with the analytical features and ingredient-class features, which are output by Model I, can be given as input to the property predicting model which maps them onto the material's chemical and/or physical properties. With the aim of optimizing the model's accuracy, and given the available dataset size, a Gaussian Process Regression (GPR) model is utilized to do so.

In case the features from Model I comprise three different feature sets (corresponding to the LR, QR-U and QR-L regressors), there will be three initial predictions per composition, received as an output in Model II. These three predictions are used in combination with cross-validation to determine a mean value and a standard deviation of the prediction, which together with the GPR uncertainty estimation provide a global uncertainty estimation for Model II.

In this context, cross-validation is performed as follows:
As is common practice in ML, the datasets are divided in a training data set (data of the polypropylene training compositions) and a test data set (data of the polypropylene test composition(s)). The test data set is kept apart and not used during the training phase (it is only used later in order to the assess the model's performance on unseen data).

From the training data set k validation data sets (each including a validation subset of the polypropylene training compositions) are split off in order to perform a k-fold cross-validation step (CV). This is done to improve the model generalization performance, prevent overfitting issues and procure an additional measure of uncertainty. Each validation data set produces a model. These models are then assembled to produce an aggregated model (denoted "GPR CV-Ensemble" in fig. 2) whose prediction value was defined as the average of the predictions from each fold. The prediction uncertainty of the model is obtained by means of adding: i) the average of the uncertainties provided by each GPR fold to ii) the standard deviation of the predictions of all folds as shown in figure 2.

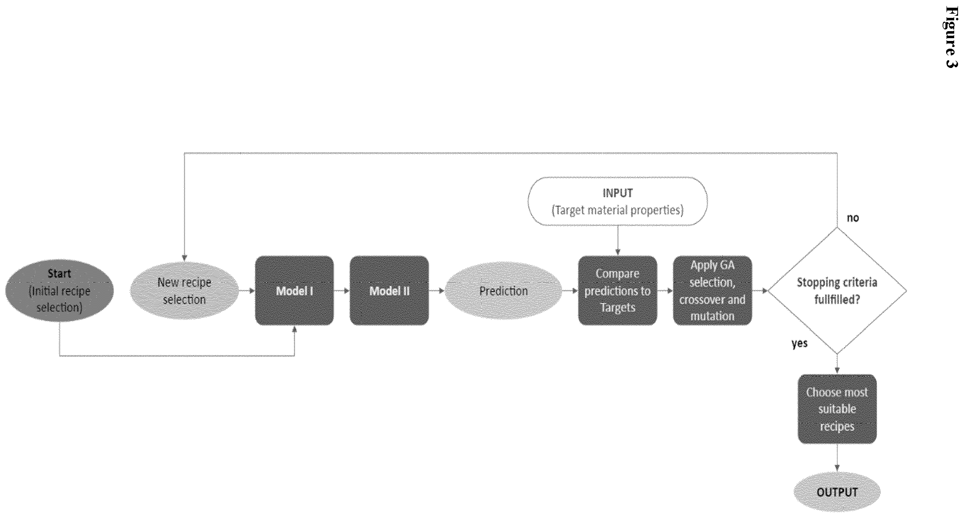

Figure 3 shows a flow diagram of Model III. If an optimization algorithm is additionally provided, as in the case of Model III, the fingerprinting model and the property prediction model can be used in reverse-engineering fashion. In this case, the user provides target material properties as input to the optimization algorithm. Model III then starts with an arbitrary initial recipe selection of a polypropylene composition and runs as many optimization loops as needed until predetermined stopping criteria are fulfilled. As an output, a selection of possible recipes that best attain the target is obtained. To this aim, a multi-objective optimization scheme is implemented using a NSGAII genetic algorithm from the Platypus Python library.

As the result of an optimization loop run, the optimization algorithm returns a "Pareto front", *i.e.* a collection of recipes, which are equally optimal. This means that, for any recommended recipe in the Pareto front, a property can only be improved by trading it off for the decrease of another property (as opposed to this, the recipes that are not in the Pareto front can still be improved without sacrificing other properties).

**Examples**

The nature of the present invention will become more clearly apparent in view of the following examples. The examples should, however, in no way limit the scope of the invention.

Determination of Analytical Features

The amorphous and crystalline fractions of the materials defined in the material space section above as well as the total ethylene weight fraction and intrinsic viscosities of the materials have been analysed by use of the CRYSTEX-QC instrument, Polymer Char (Valencia, Spain). Details of the technique and the method can be found in literature (Ljiljana Jeremic, Andreas Albrecht, Martina Sandholzer & Markus Gahleitner (2020) Rapid characterization of high-impact ethylene-propylene copolymer composition by crystallization extraction separation: comparability to standard separation methods, International Journal of Polymer Analysis and Characterization, 25:8, 581-596).

An initial material solution was prepared by adding 1,2,4-trichlorobenzene (TCB) containing 250 mg 1,2,6-tert-butyl-4-methylphenol (BHT) as antioxidant to the material and by stirring at 400 rpm and 160 °C until complete dissolution was achieved, usually for about 60 min. To avoid degradation, the polymer solution was blanketed with the $N_2$ atmosphere during dissolution.

Afterwards the initial material solution was crystallised. A defined volume of the sample solution was injected into a column which is kept at 40 °C and which was filled with inert support so that the crystallization of the material and separation of the soluble fraction from the crystalline part was taking place. This process was repeated two times.

The content of the amorphous fraction (sol) was determined by measuring the concentration of the initial material solution and the concentration of the solution after crystallization by means of an IR4 detector. Furthermore, the IR4 detector was used for the determination of the total ethylene weight fraction (c2).

For the determination of intrinsic viscosity an online 2-capillary viscometer was used.

The IR4 detector was a multiple wavelength detector measuring IR absorbance at two different bands ($CH_3$ stretching vibration (centred at app. 2960 $cm^{-1}$) and the CH stretching vibration (2700-3000 $cm^{-1}$). The IR4 detector was calibrated with a series of 8 EP copolymers with known ethylene content in the range of 2 wt.-% to 69 wt.-% (determined by $^{13}$C-NMR) and each at various concentrations, in the range of 2 and 13 mg/ml. To account for both features, content and total ethylene weight fraction, at the same time, the following calibration equations were applied:

$$\text{Content} = a + b*\text{Abs(CH)} + c*(\text{Abs(CH)})^2 + d*\text{Abs}(CH_3) + e*(\text{Abs}(CH_3)^2 + f*\text{Abs(CH)}*\text{Abs}(CH_3) \qquad \text{(equation 1)}$$

$$CH_3/1000C = a + b*\text{Abs(CH)} + c*\text{Abs}(CH_3) + d*(\text{Abs}(CH_3)/\text{Abs(CH)}) + e*(\text{Abs}(CH_3)/\text{Abs(CH)})^2 \qquad \text{(equation 2)}$$

The constants a to e for equation 1 and a to f for equation 2 were determined by using least square regression

analysis.

The $CH_3/1000C$ was converted to the total ethylene weight fraction in wt.-% using following relationship:

$$\text{wt.-\% (Ethylene in EP Copolymers)} = 100 - CH_3/1000C * 0.3 \quad \text{(equation 3)}$$

Content of amorphous fraction (sol) and crystalline fraction (cr) were correlated through a XS-calibration to the "Xylene Cold Soluble" (XCS) quantity and respectively "Xylene Cold Insoluble" (XCI) fractions, determined according to standard gravimetric method as per ISO16152. XS-calibration was achieved by testing various EP copolymers with XCS content in the range 2-31 wt.-%. The determined XCS calibration is linear:

$$\text{wt.-\% XCS} = 1.01 * \text{wt.-\% sol} \quad \text{(equation 4)}$$

Intrinsic viscosity (iv) of the material and intrinsic viscosity of its amorphous fraction (ivsol) as well as intrinsic viscosity of its crystalline fraction (ivcr) were determined by use of an online 2-capillary viscometer and can be correlated to corresponding iv's determined by standard method in decalin according to ISO 1628-3. Calibration was achieved with various propylene copolymers having an iv in the range of 2-4 dL/g. The determined calibration curve is linear:

$$\text{iv (dL/g)} = a * Vsp/c \quad \text{(equation 5)}$$

Training and Performance of Fingerprinting Model

The fingerprinting model has been trained with a database comprising information on contents of ingredients and values for the analytical features of several polypropylene training compositions. The values for the analytical features have been obtained as described above.

The performance of the trained fingerprinting model was quantified for both, polypropylene training compositions and polypropylene test compositions. The coefficient of determination ($R^2$) and the mean absolute error (MAE) are indicated in table 2 below.

*Table 2*

| feature | $R^2$ | MAE |
|---|---|---|
| sol | 0.98 | 0.05 |
| c2 | 0.98 | 0.08 |
| c2cr | 0.89 | 0.14 |
| c2sol | 0.92 | 0.13 |
| iv | 0.98 | 0.01 |
| ivcr | 0.98 | 0.02 |
| ivsol | 0.92 | 0.07 |
| ivratio | 0.93 | 0.07 |

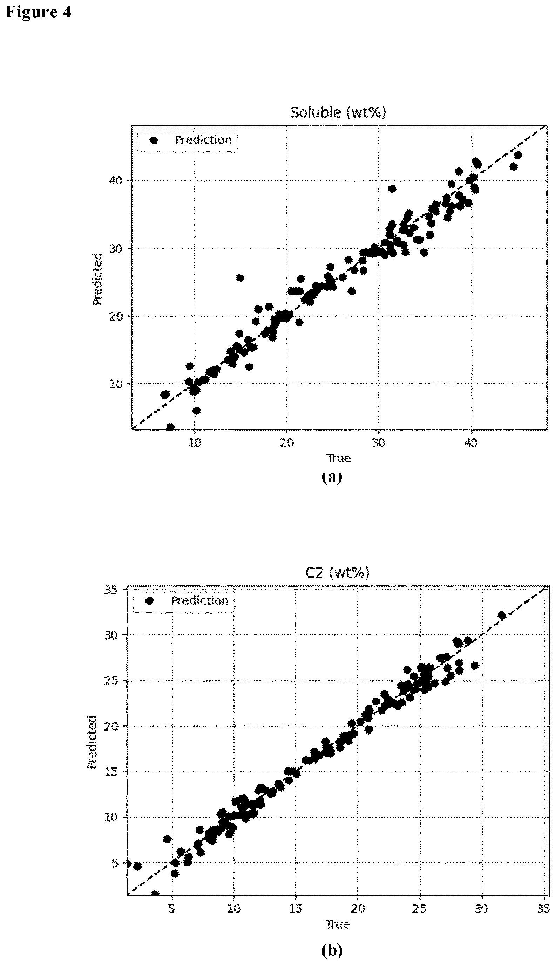

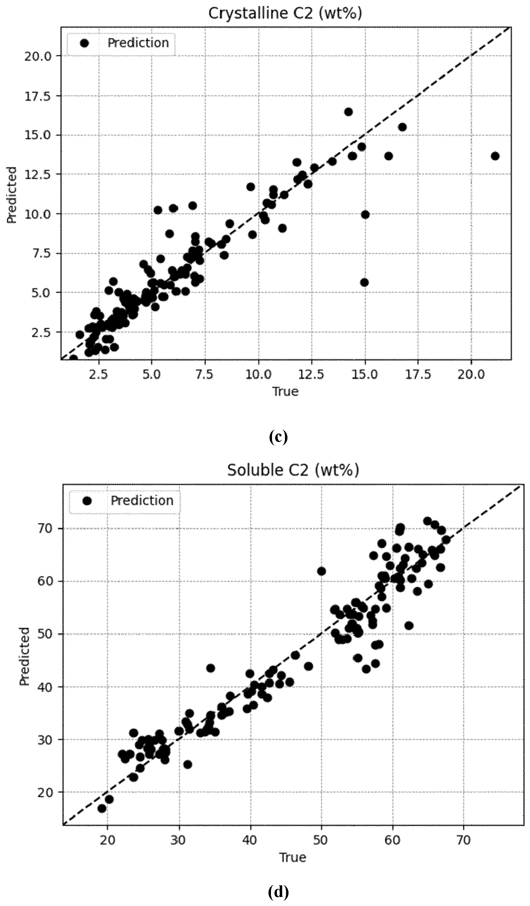

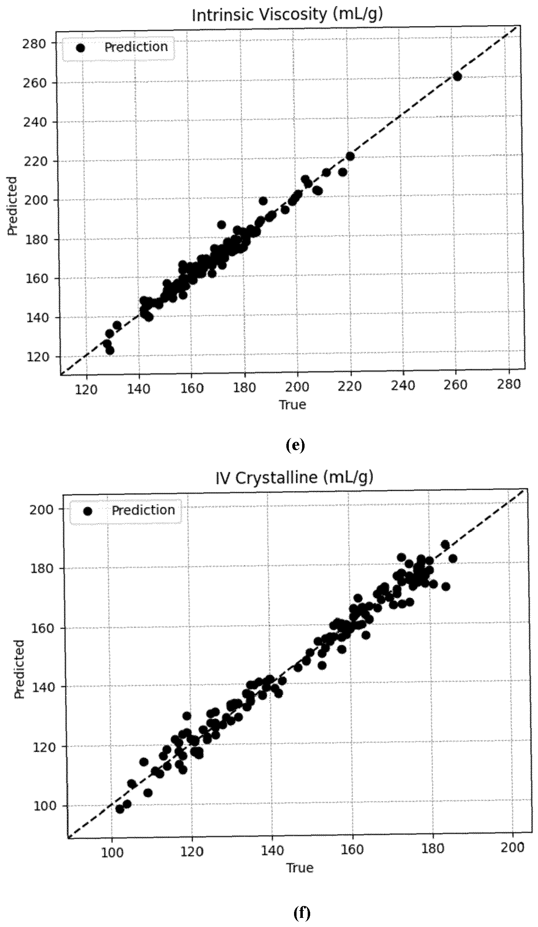

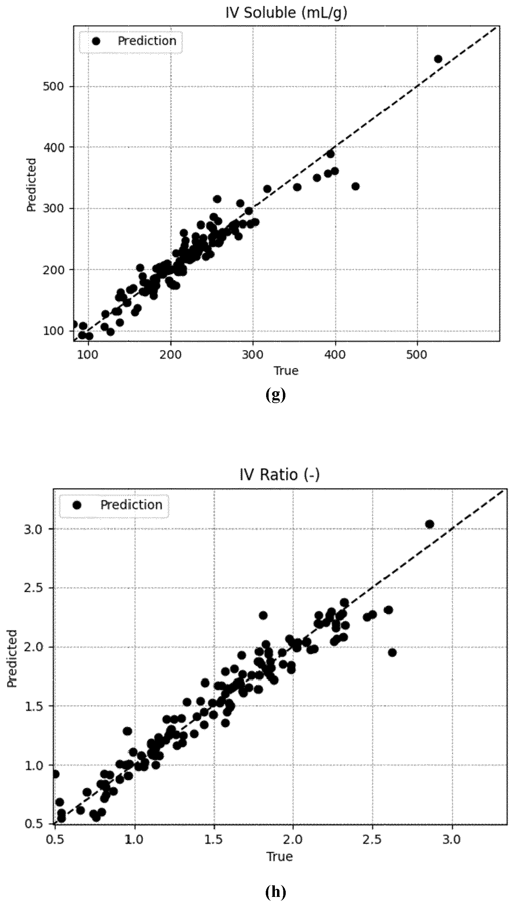

The corresponding prediction accuracy plots are shown in Figure 4 (a) - (h). The plots allow to see how much variance is in the model by comparing the location of the predicted properties against the 45° dashed line. If the points would come to lie on the 45° dashed line, the prediction would exactly match the experimentally measured value, which is assumed as being the "true" value.

Determination of Chemical and Physical Properties

*Young's Modulus (E-23, E-m30):*

was measured at +23 °C or -30 °C according to ISO 527-1:2012/ISO527-2:2012 and at a cross head speed of 1 mm/min using injection moulded specimens as described in EN ISO 1873-2 (dog bone shape, 4 mm thickness).

*Yield Stress (s-23, s-m30):*

was measured according to ISO 527-1:2012/ISO527-2:2012 at +23 °C or -30°C and at a cross head speed of 50 mm/min using injection moulded specimens as described in EN ISO 1873-2 (dog bone shape, 4 mm thickness).

*Notched Charpy impact strength (charpy-23, charpy-m20):*

was measured according to ISO 179-2:2020 at +23 °C and -20 °C, using injection moulded bar test specimens of $80 \times 10 \times 4$ mm$^3$ prepared in accordance with ISO 294-1:1996.

*Impact puncture energy (ipt-23, ipt-m30):*

was determined in the instrumented falling weight test according to ISO 6603-2 at test temperatures of +23 °C and -30 °C using an injection moulded plaque of $60 \times 60 \times 2$ mm. The reported puncture energy results from an integral of the force-displacement curve.

*Coefficient of linear thermal expansion (CTLE-TD, CTLE-MD):*

was determined in accordance with ISO 11359-2:1999 on 10 mm long pieces cut from the same injection moulded specimens as used for the determination for Young's Modulus. The measurement was performed in a temperature range from -30 to +80 °C at a heating rate of 1 °C/min and a temperature range from 23 to 80 °C at a heating rate of 1 °C/min in machine direction or in transverse direction, respectively.

*Melt flow rate (MFR):*

was determined as the quantity of polymer in grams which the test apparatus standardized to ISO 1133 extrudes within 10 minutes at a temperature of 230 °C under a load of 2.16 kg.

*Shrinkage in flow (shri_sec_avg_flow) and shrinkage cross flow (shri_sec_avg_flow)* were determined on film gate injection moulded discs: One was a sector (radius 300 mm and opening angle of 20 °) and the other one a stripe (340x65 mm). 2.8 mm thick specimen were injection moulded at the same time at a back pressure of 400 bar. The melt temperature was 240 °C and the temperature of the tool 25 °C. Average flow front velocity was 3.5 $\pm$ 0.2 mm/s for the 2.8 mm tool. After the injection moulding process the shrinkage of the specimens was measured at 23 °C and 50 % humidity. The measurement interval was 96 hours after the injection moulding. To determine the shrinkage 83 and 71 measurement points (generated by eroded dots on the tool surface) of the sector and the stripe, respectively, were recorded with a robot. At least the in flow shrinkage of the 2.8 mm thick plates exposed to a back pressure of 400 bars at 96 hours after the injection moulding process was reported as final result.

*Tiger stripes (mse-3):*

The tendency to show tiger stripes (MSE) or flow marks was examined with a method as described below. This method is disclosed in detail in WO 2010/149529, which is incorporated herein in its entirety.

An optical measurement system, as described by Sybille Frank et al. in PPS 25 Intern. Conf. Polym. Proc. Soc 2009 or Proceedings of the SPIE, Volume 6831, pp 68130T-68130T-8 (2008) was used for characterizing the surface quality.

This method consists of two aspects:

> 1. Image recording:
> The basic principle of the measurement system is to illuminate the plates with a defined light source (LED) in a closed environment and to record an image with a CCD-camera system.
> 2. Image analysis:
> The specimen is floodlit from one side and the upwards reflected portion of the light is deflected via two mirrors to a CCD-sensor. The such created grey value image is analyzed in lines. From the recorded deviations of grey values the mean square error (MSE) is calculated allowing a quantification of surface quality, i.e. the larger the MSE value the more pronounced is the surface defect.

Generally, for one and the same material, the tendency to flow marks increases when the injection speed is increased.

For the evaluation plaques 440x148x2.8 mm with grain VW K50 and a Hot I gate of 2.8 mm were used. The plaques were produced with filling time of 3.0 s.

Further conditions:

Melt temperature = 240 °C

Mould temperature = 30 °C

Dynamic pressure = 10 bar hydraulic

The smaller the MSE value is at a certain filling time, the smaller is the tendency for flow marks.

Determination of recyclate properties

*Limonene detection:* Limonene is a substance typically detected in post-consumer recyclates originating from household waste streams. Thus, it is used as an identification feature for the recyclates. Limonene quantification can be carried out using solid phase microextraction (HS-SPME-GC-MS) by standard addition.

50 mg ground samples were weighed into 20 mL headspace vials and after the addition of limonene in different concentrations and a glass-coated magnetic stir bar, the vial was closed with a magnetic cap lined with silicone/PTFE. Micro capillaries (10 pL) were used to add diluted limonene standards of known concentrations to the sample. Addition of 0, 2, 20 and 100 ng equals 0 mg/kg, 0.1 mg/kg, 1mg/kg and 5 mg/kg limonene, in addition standard amounts of 6.6 mg/kg, 11 mg/kg and 16.5 mg/kg limonene was used in combination with some of the samples tested in this application. For quantification, ion-93 acquired in SIM mode was used. Enrichment of the volatile fraction was carried out by headspace solid phase microextraction with a 2 cm stable flex 50/30 pm DVB/Carboxen/PDMS fibre at 60 °C for 20 minutes. Desorption was carried out directly in the heated injection port of a GCMS system at 270 °C.

GCMS Parameters:

Column: 30 m HP 5 MS 0.25*0.25

Injector: Splitless with 0.75 mm SPME Liner, 270°C

Temperature program: -10 °C (1 min)

Carrier gas: Helium 5.0, 31 cm/s linear velocity, constant flow

MS: Single quadrupole, direct interface, 280 °C interface temperature

Acquisition: SIM scan mode

Scan parameter: 20-300 amu

SIM Parameter: m/Z 93, 100 ms dwell time

*Black spot concentration:* Each post-consumer recycled polymer resin contains a certain level of contaminants which can be quantified by the method described here as "Black spot contamination". Digital images of 5 injection moulded plaques were collected with a Canon EOS R6 camera. Settings were fixed such that a resolution of about 15 pixel/mm were obtained. Once the image had been converted to grey scale and the intensity normalized between 0 and 256, the exposure time was fixed such that a grey level of ca. 160 was obtained for the plaques.

Image analysis was performed with the Wavemetrics IgorPro software. A Region of Interest area was fixed inside the plaques, with size of $2100 \times 1125$ pixels$^2$. A Prewitt edge detection algorithm, set with threshold of 10, was used to identify the particles on the surface. The edge image was then analysed to gather information about the number and size of particles detected, with a minimum area of 5 pixels and excluding particles touching the border of the area analysed. This operation was repeated for the 5 plaques imaged, and a size distribution of particles was created. The number of particles was normalized by the total area imaged in the 5 plaques, and therefore a frequency of particles as #/cm$^2$ was obtained. The particles were grouped in bins according to their size, with bin size of 0.05 mm$^2$.

*CIELAB color space (L\*a\*b\*):* In the CIE L\*a\*b\* uniform color space, the color coordinates are: L\*-the lightness coordinate; a\*-the red/green coordinate, with +a\* indicating red, and -a\* indicating green; and b\*-the yellow/blue coordinate, with +b\* indicating yellow, and - b\* indicating blue. The L\*, a\*, and b\*coordinate axis define the three-dimensional CIE color space. Standard Konica/Minolta Colorimeter CM-3700A.

*Inorganic residues:* Inorganic residues were quantified according to DIN ISO 1172:1996 using a Perkin Elmer TGA 8000. Approximately 10-20 mg of material was placed in a platinum pan. The temperature was equilibrated at 50 °C for 10 minutes, and afterwards raised to 950 °C under nitrogen at a heating rate of 20 °C/min. The ash content was evaluated as the weight % at 850°C.

Training and Performance of Property Predicting Model

The property predicting model was trained using the analytical features, the contents of ingredients (i.e. the "ingredient-class features vector", as exemplified in fig. 1), and the chemical and physical properties, measured as described above, for the materials as defined in the material space section.

Table 3 shows the prediction performance of the property prediction model for a validation subset of polypropylene training compositions and polypropylene test compositions. Here, it is important to recall that the validation subset of polypropylene training compositions allows to quantify how good the model fits the observed data, whereas the polypropylene test compositions reveals how good the model is able to predict previously unseen data.

*Table 3*

| Target | Valid. MAE | Valid. $R^2$ | Test MAE | Test $R^2$ |
|---|---|---|---|---|
| E-23 | 0.03 | 0.99 | 0.07 | 0.90 |
| E-m30 | 0.04 | 0.97 | 0.05 | 0.92 |
| s-23 | 0.00 | 1.00 | 0.02 | 0.96 |
| s-m30 | 0.03 | 0.95 | 0.05 | 0.90 |
| CLTE-TD | 0.02 | 1.00 | 0.13 | 0.97 |
| CLTE-MD | 0.04 | 0.97 | 0.07 | 0.93 |
| MFR | 0.01 | 0.99 | 0.02 | 0.96 |
| shri_sec_avg_flow | 0.06 | 0.96 | 0.07 | 0.92 |
| charpy-23 | 0.00 | 1.00 | 0.21 | 0.90 |
| charpy-m20 | 0.00 | 1.00 | 0.27 | 0.92 |
| ipt-23 | 0.30 | 0.93 | 0.41 | 0.70 |
| ipt-m30 | 0.69 | 0.93 | 0.74 | 0.92 |
| mse-3 | 0.11 | 0.96 | 0.34 | 0.73 |

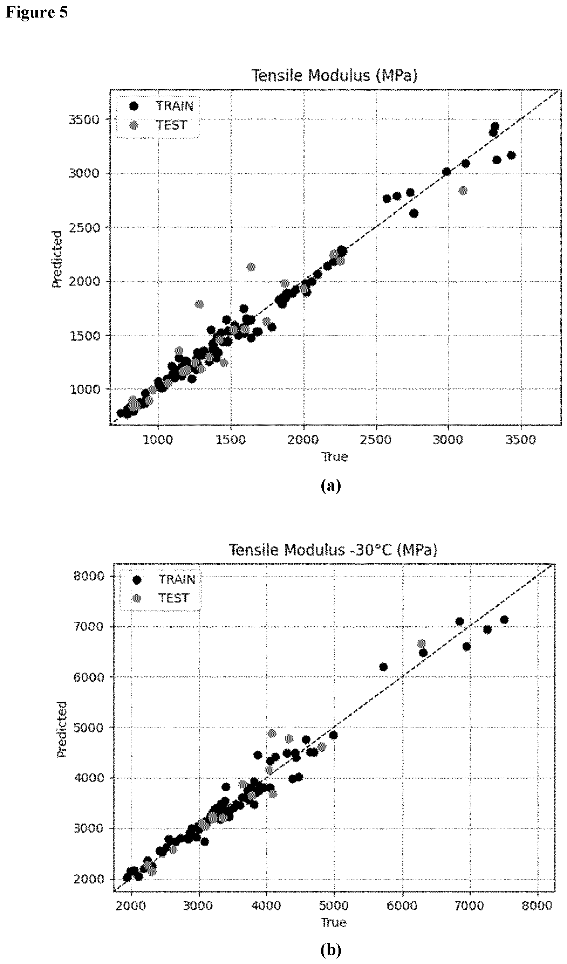

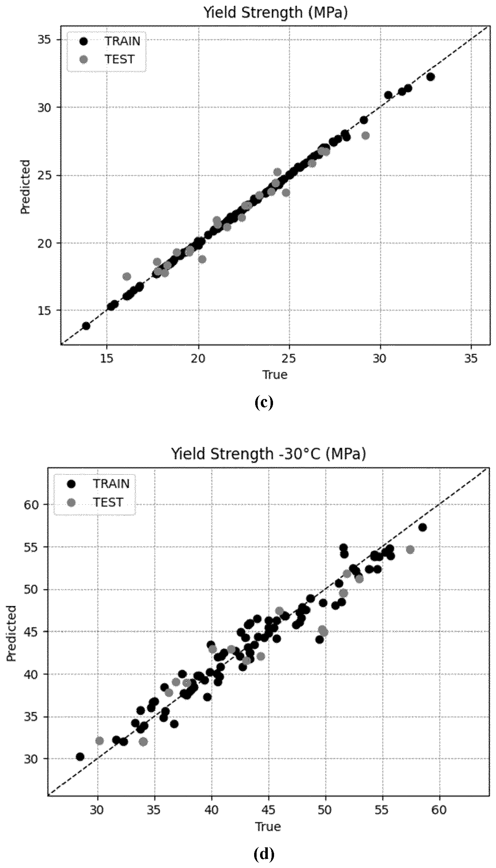

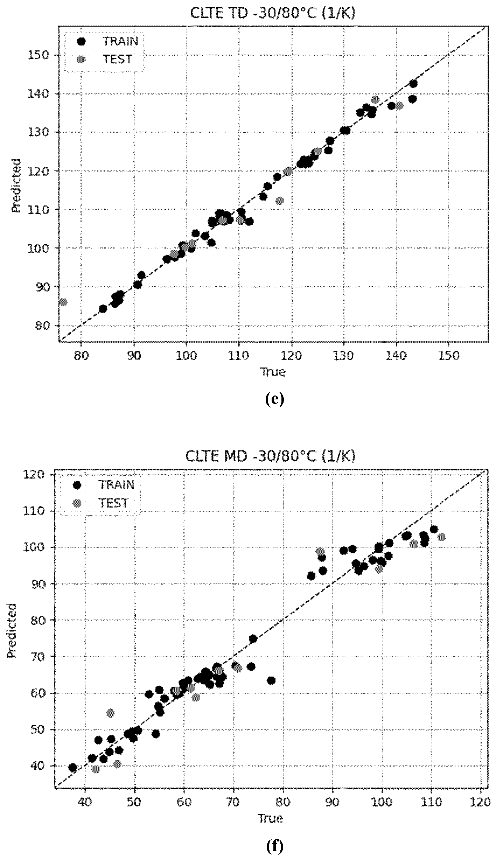

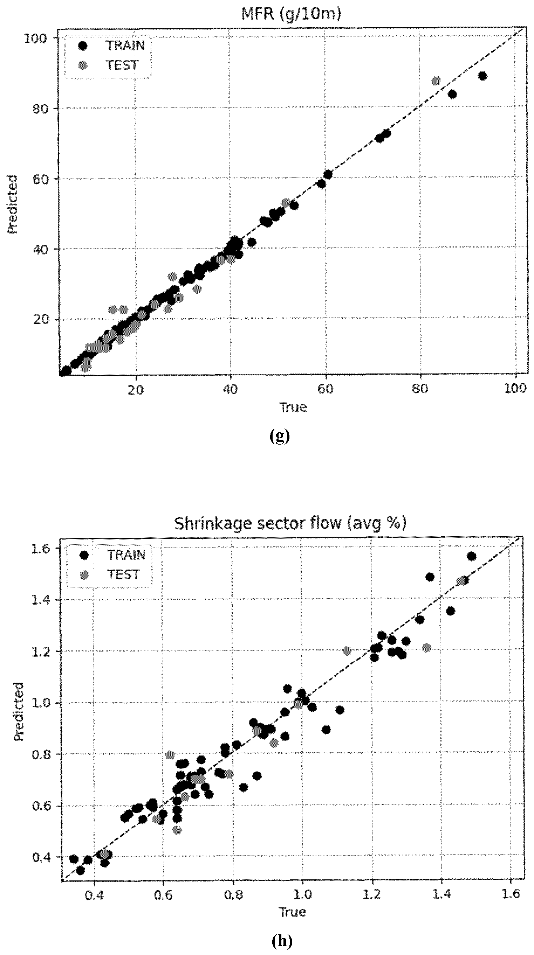

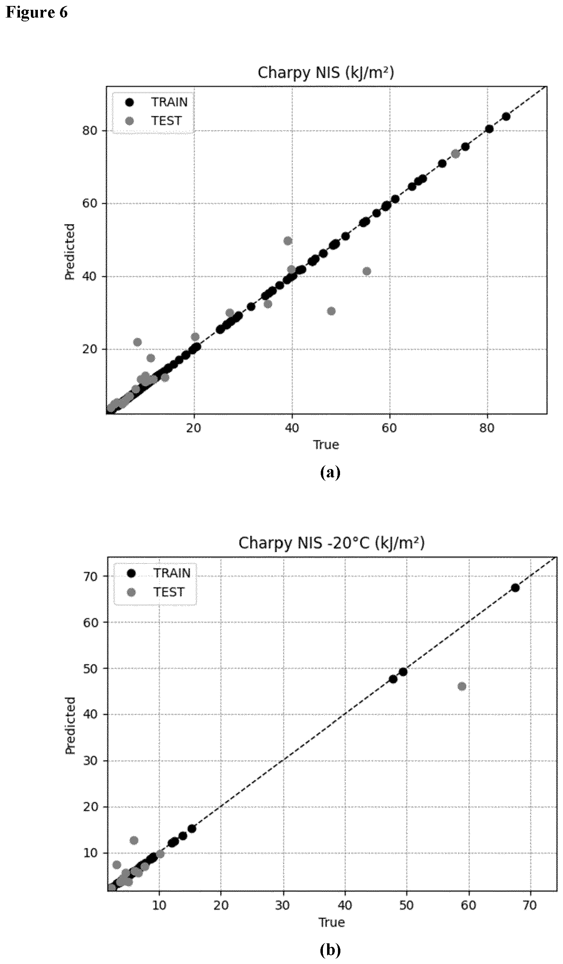

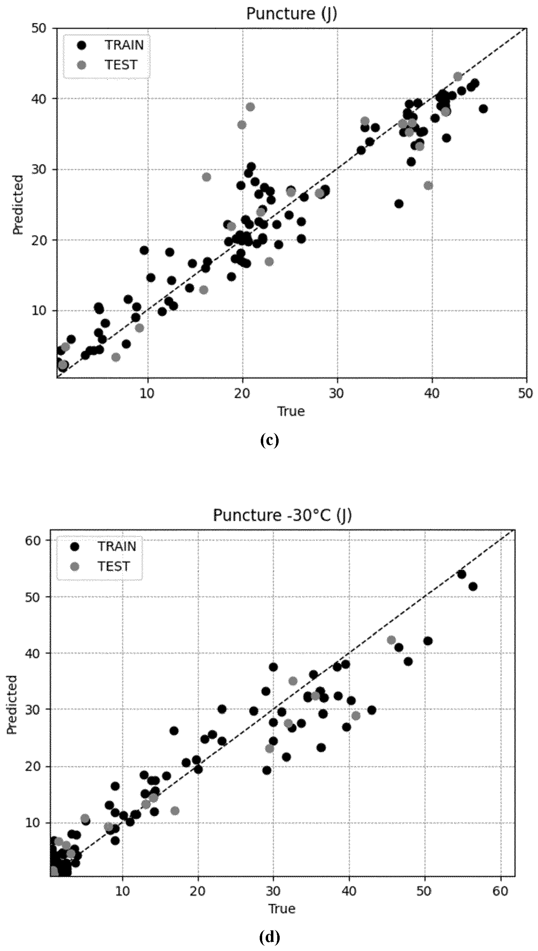

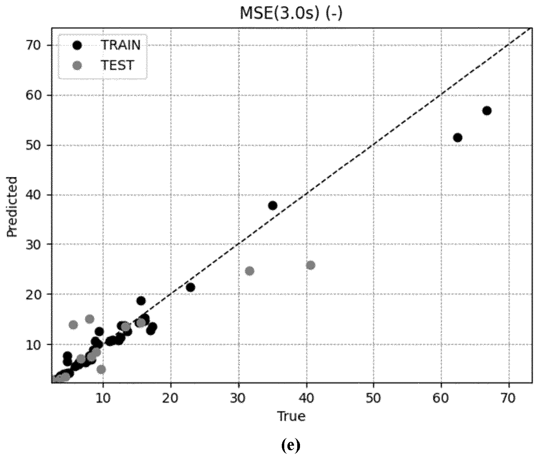

The prediction performance for the validation subset of polypropylene training compositions is notably good for all properties, with a minimum value being 0.93 and a maximum value being 1.00. The prediction performance for the polypropylene test compositions is equally good with the exception of the impact variables (Charpy and IPT) where the performance was lower, however still acceptable. Figure 5 (a) - (h) and Figure 6 (a) - (e) show the corresponding prediction accuracy plots, whereby predictions for the validation subset of polypropylene training compositions are shown in black and predictions for the polypropylene test compositions are shown in grey.

Performance of Model III

Model III uses a multi-objective optimization approach to search for recipes fulfilling specific target properties. For this example, stiffness at 23°C (E-23 = 1000 MPa), melt flow rate (MFR = 20 g/10m) and Charpy impact strength at 23°C (charpy-23 = 25 kJ/m²) have been chosen as target properties. Based on a genetic algorithm for the multi-objective optimization, Model III runs as many loops as needed until a stopping criterion is satisfied. In this way, it is iteratively computing a "Pareto front" (the set of non-dominated solutions) and returning a collection of recipes which are optimal in view of the constraints (here, the values that have been input for the target properties regarding E-23, MFR and charpy-23). Since the Pareto front is, by definition, a set of optimum solutions, normally a decision is still required in order to choose the most satisfactory solution from this set. In this case, model III has been implemented to suggest the "knee-point" of the Pareto front (that is, the point that minimizes the overall difference between predicted and target properties) as a recipe candidate. The other points in the Pareto front provide different trade-offs where a target can be improved at the expense of a reduction in one or more of the other target variables.

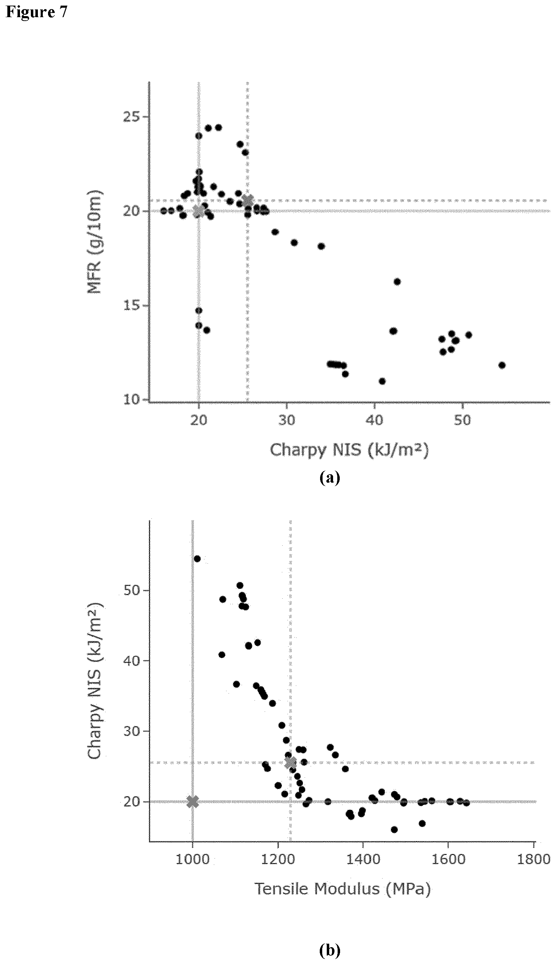

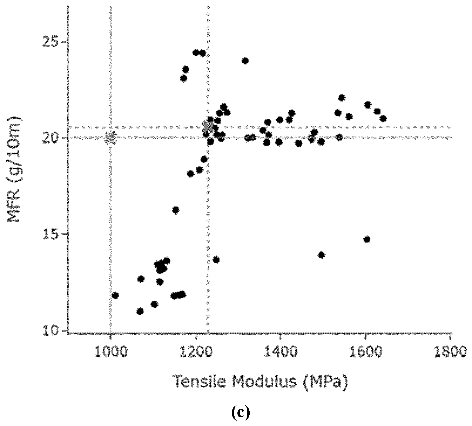

Figure 7 (a), (b) and (c) show the final Pareto front projected in the three different possible planes for the three target variables (i.e. Charpy vs. MFR, Stiffness vs. Charpy and Stiffness vs. MFR). In this case, a recipe containing 42 wt.-% of a heterophasic propylene copolymer, 38 wt.-% of a reactor grade heterophasic propylene copolymer, 12 wt.-% of a polyolefin elastomer as an impact modifier and 5 wt.-% of a talc as filler is recommended by model III. In Figure 7, this recipe will have the properties that are indicated by the point defined by the dashed-line cross. Likewise, the point defined by the solid-line cross shows the target properties that were input into the model.

This example shows clearly how other recipe candidates exist in the Pareto front where different trade-offs are possible (for instance, a higher stiffness and Charpy energy would be possible, however at the cost of reducing the MFR).

**Claims**

1. A computer-implemented method for predicting attributes of a polypropylene composition, the method comprising the following steps:

   Ia) training a fingerprinting model with polypropylene training compositions by mapping contents of ingredients of the polypropylene training compositions to analytical features of the polypropylene training compositions;
   Ib) feeding the trained fingerprinting model, as an input, with contents of ingredients of a polypropylene test composition; and
   Ic) receiving, as an output, analytical features of the polypropylene test composition,

   wherein the analytical features comprise at least one feature characteristic of the amorphous fraction or the crystalline fraction of the polypropylene composition.

2. The computer-implemented method according to claim 1, wherein the polypropylene training compositions and the polypropylene test composition comprise at least one polypropylene, wherein the at least one polypropylene is preferably selected from the group consisting of propylene homopolymer, random propylene copolymer, heterophasic propylene copolymer and combinations or blends thereof.

3. The computer-implemented method according to one of claims 1 and 2, wherein the polypropylene training compositions and/or the polypropylene test composition comprise(s) at least one further ingredient selected from the group consisting of:

   - impact modifier,
   - filler, and
   - polyethylene.

4. The computer-implemented method according to any of the preceding claims, wherein the at least one feature characteristic of the amorphous fraction or the crystalline fraction is selected from the group consisting of:

   - an amorphous fraction percentage,
   - a crystalline fraction percentage,
   - a ratio of amorphous fraction percentage : crystalline fraction percentage,
   - an ethylene content in the amorphous fraction,
   - an ethylene content in the crystalline fraction,
   - a ratio of ethylene content in the amorphous fraction : ethylene content in the crystalline fraction,
   - an intrinsic viscosity in the amorphous fraction,
   - an intrinsic viscosity in the crystalline fraction, and
   - a ratio of intrinsic viscosity in the amorphous fraction : intrinsic viscosity in the crystalline fraction.

5. The computer-implemented method according to any of the preceding claims, wherein the analytical features further comprise at least one of a total ethylene weight fraction and an intrinsic viscosity of the polypropylene composition.

6. The computer-implemented method according to any of the preceding claims, wherein the fingerprinting model is a regression-based algorithm,
   wherein the regression-based algorithm preferably comprises a linear regression and, optionally, a quantile regression.

7.  The computer-implemented method according to any of the preceding claims, further comprising the following steps:

    IIa) training a property predicting model with polypropylene training compositions by mapping analytical features and at least contents of non-polymeric ingredients of polypropylene training compositions to at least one property of the polypropylene training compositions;
    IIb) feeding the trained property predicting model, as an input, with analytical features of the polypropylene test composition as received in step Ic) and at least contents of non-polymeric ingredients of the polypropylene test composition,
    IIc) receiving, as an output, at least one property of the polypropylene test composition,

    wherein the at least one property is selected from the group consisting of chemical and physical properties.

8.  The computer-implemented method according to any of the preceding claims, wherein the chemical and physical properties are selected from the group consisting of mechanical properties, rheological properties, processing properties and surface properties,
    wherein the chemical and physical properties preferably comprise at least one of the following properties:

    - Young's modulus, preferably at room temperature or at -30 °C;
    - Yield stress, preferably at room temperature or at -30 °C;
    - Notched Charpy impact strength, preferably at room temperature or at -20 °C;
    - Impact puncture energy, preferably at room temperature or at -30 °C;
    - Coefficient of linear thermal expansion, preferably in flow direction or in transverse direction;
    - Melt flow rate, preferably at 2.16 kg and 230 °C or at 21.6 kg and 230 °C;
    - Shrinkage, preferably after injection in the flow direction and/or perpendicular to the flow direction;
    - a value indicative of tiger stripes, preferably after production of an injection mould with a filling time of 3.0 s; and scaled versions of the aforementioned properties, more preferably scaled versions obtained after a hyperbolic-tangent transformation or a logarithmic transformation.

9.  The computer-implemented method according to one of claims 7 and 8, wherein the property predicting model is a Gaussian Process Regression (GPR).

10. The computer-implemented method according to one of claims 7 to 9, further comprising an optimization algorithm,

    preferably an optimization algorithm for predicting at least one recipe of a polypropylene composition which comes closest to at least one predetermined target property,
    more preferably a genetic algorithm for predicting at least one recipe of a polypropylene composition which comes closest to at least one predetermined target property.

11. The computer-implemented method according to claim 10, wherein the optimization algorithm comprises the following steps:

    IIIa) receiving at least one target property as an input,
    IIIb) comparing the at least one property of the polypropylene test composition as received in step IIc) with the at least one target property, and
    IIIc) when the comparison fulfils a predetermined stopping criterion, receiving, as an output, the contents of ingredients of the polypropylene test composition in the form of a recipe.

12. The computer-implemented method according to one of claims 10 and 11, wherein the optimization algorithm is a non-dominated sorting based multi-objective evolutionary algorithm (NSGAII) receiving more than one target property as an input.

13. The method of one of the preceding claims, wherein the output is used for controlling a process in a development of application-tailored polypropylene composition.

14. A non-transitory computer readable storage medium for tangibly storing computer program instructions capable of being executed by a processor, the computer program instructions defining the steps of one of the preceding method claims.

**15.** Use of analytical features comprising at least one feature characteristic of the amorphous fraction or the crystalline fraction of a polypropylene composition and contents of ingredients of polypropylene compositions for training a computer-implemented model.

**Figure 1**

**Figure 2**

EP 4 456 077 A1

Figure 3

Start (Initial recipe selection) → New recipe selection → Model I → Model II → Prediction → Compare predictions to Targets → Apply GA selection, crossover and mutation → Stopping criteria fullfilled?

INPUT (Target material properties)

no

yes → Choose most suitable recipes → OUTPUT

**Figure 4**

(a)

(b)

Crystalline C2 (wt%)

(c)

Soluble C2 (wt%)

(d)

(e)

(f)

IV Soluble (mL/g)

**(g)**

IV Ratio (-)

**(h)**

**Figure 5**

Tensile Modulus (MPa)

(a)

Tensile Modulus -30°C (MPa)

(b)

**(c)**

**(d)**

CLTE TD -30/80°C (1/K)

**(e)**

CLTE MD -30/80°C (1/K)

**(f)**

**(g)**

**(h)**

**Figure 6**

(a)

(b)

**(c)**

**(d)**

(e)

**Figure 7**

(a)

(b)

**(c)**

Europäisches
Patentamt

European
Patent Office

Office européen
des brevets

## EUROPEAN SEARCH REPORT

**Application Number**

**EP 23 17 0271**

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ANDRAJU N. ET AL: "Machine-Learning-Based Predictions of Polymer and Postconsumer Recycled Polymer Properties: A Comprehensive Review", APPLIED MATERIALS & INTERFACES, vol. 14, no. 38, 14 September 2022 (2022-09-14), pages 42771-42790, XP093087534, US ISSN: 1944-8244, DOI: 10.1021/acsami.2c08301 Retrieved from the Internet: URL:https://pubs.acs.org/doi/pdf/10.1021/acsami.2c08301> * the whole document * | 1-15 | INV. G16C20/30 ADD. G16C20/70 G16C60/00 |
| A | WANG J. ET AL: "Estimating the Relative Crystallinity of Biodegradable Polylactic Acid and Polyglycolide Polymer Composites by Machine Learning Methodologies", POLYMERS, vol. 14, no. 3, 28 January 2022 (2022-01-28), page 527, XP093087527, DOI: 10.3390/polym14030527 * the whole document * | 1-15 | |
|  | -/-- | | TECHNICAL FIELDS SEARCHED (IPC) G16C |

The present search report has been drawn up for all claims

| Place of search | Date of completion of the search | Examiner |
|---|---|---|
| The Hague | 2 October 2023 | Godzina, Przemyslaw |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## EUROPEAN SEARCH REPORT

Application Number

EP 23 17 0271

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | ISHIKIRIYAMA K.: "Polymer informatics based on the quantitative structure-property relationship using a machine-learning framework for the physical properties of polymers in the ATHAS data bank", THERMOCHIMICA ACTA, ELSEVIER SCIENCE PUBLISHERS, AMSTERDAM, NL, vol. 708, 25 December 2021 (2021-12-25), XP086927605, ISSN: 0040-6031, DOI: 10.1016/J.TCA.2021.179135 [retrieved on 2021-12-25] * the whole document * * in particular * ----- | 1-15 | |
| A | RAMPRASAD R. ET AL: "Machine learning in materials informatics: recent applications and prospects", COMPUTATIONAL MATERIALS, vol. 3, no. 1, 13 December 2017 (2017-12-13), pages 1-13, XP093087029, DOI: 10.1038/s41524-017-0056-5 Retrieved from the Internet: URL:https://www.nature.com/articles/s41524-017-0056-5> * the whole document * ----- | 1-15 | TECHNICAL FIELDS SEARCHED (IPC) |

-/--

The present search report has been drawn up for all claims

| Place of search | Date of completion of the search | Examiner |
|---|---|---|
| The Hague | 2 October 2023 | Godzina, Przemyslaw |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 23 17 0271

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | CHEN L. ET AL: "Polymer informatics: Current status and critical next steps", MATERIALS SCIENCE AND ENGINEERING: R: REPORTS, vol. 144, 26 December 2020 (2020-12-26), page 100595, XP093087333, AMSTERDAM, NL ISSN: 0927-796X, DOI: 10.1016/j.mser.2020.100595 * the whole document * | 1-15 | |
| A | KIM C. ET AL: "Polymer Genome: A Data-Powered Polymer Informatics Platform for Property Predictions", THE JOURNAL OF PHYSICAL CHEMISTRY C, vol. 122, no. 31, 13 July 2018 (2018-07-13), pages 17575-17585, XP093087044, US ISSN: 1932-7447, DOI: 10.1021/acs.jpcc.8b02913 * the whole document * | 1-15 | |
| A | KIM C. ET AL: "Polymer design using genetic algorithm and machine learning", COMPUTATIONAL MATERIALS SCIENCE, ELSEVIER, AMSTERDAM, NL, vol. 186, 28 September 2020 (2020-09-28), XP086362619, ISSN: 0927-0256, DOI: 10.1016/J.COMMATSCI.2020.110067 [retrieved on 2020-09-28] * the whole document * | 1-15 | TECHNICAL FIELDS SEARCHED (IPC) |

-/--

The present search report has been drawn up for all claims

| Place of search | Date of completion of the search | Examiner |
|---|---|---|
| The Hague | 2 October 2023 | Godzina, Przemyslaw |

EPO FORM 1503 03.82 (P04C01)

Europäisches
Patentamt

European
Patent Office

Office européen
des brevets

# EUROPEAN SEARCH REPORT

Application Number

**EP 23 17 0271**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | CENCER M. M. ET AL: "Machine learning for polymeric materials: an introduction", POLYMER INTERNATIONAL, vol. 71, no. 5, 4 December 2021 (2021-12-04), pages 537-542, XP093035941, GB ISSN: 0959-8103, DOI: 10.1002/pi.6345 Retrieved from the Internet: URL:https://onlinelibrary.wiley.com/doi/full-xml/10.1002/pi.6345> * the whole document * * in particular * * par. POLYMER REPRESENTATION AND FEATURIZATION * * figure 3 * ----- | 1-15 | |

TECHNICAL FIELDS
SEARCHED (IPC)

The present search report has been drawn up for all claims

| Place of search | Date of completion of the search | Examiner |
|---|---|---|
| The Hague | 2 October 2023 | Godzina, Przemyslaw |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
...............................................................................
& : member of the same patent family, corresponding document

EPO FORM 1503 03.82 (P04C01)

page 4 of 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 20220199971 A1
- US 10955362 B2
- EP 0887379 A
- WO 9212182 A
- WO 2004000899 A
- WO 2004111095 A
- WO 9924478 A
- WO 9924479 A
- WO 0068315 A
- WO 2010149529 A

### Non-patent literature cited in the description

- *Pure Appl. Chem.,* 1996, vol. 68 (8), 1591-1595
- **DEL HIERRO, P. ; ORTIN, A. ; MONRABAL, B.** Soluble Fraction Analysis in polypropylene. *The Column,* February 2014
- **PEDREGOSA et al.** *Gaussian Processes*
- **LJILJANA JEREMIC ; ANDREAS ALBRECHT ; MARTINA SANDHOLZER ; MARKUS GAHLEITNER.** Rapid characterization of high-impact ethylene-propylene copolymer composition by crystallization extraction separation: comparability to standard separation methods. *International Journal of Polymer Analysis and Characterization,* 2020, vol. 25 (8), 581-596
- **SYBILLE FRANK et al.** PPS 25 Intern. *Conf. Polym. Proc. Soc,* 2009
- *Proceedings of the SPIE,* 2008, vol. 6831, 68130T-68130T, 8